# EUROPEAN PATENT APPLICATION

(11) **EP 2 995 249 A1**
(43) Date of publication of application: **16.03.2016**
(21) Application number: 13884183.8
(22) Date of filing: 10.05.2013
(51) Int. Cl.: A61B 5/0245

(54) **DIAGNOSTIC DEVICE, DIAGNOSTIC METHOD AND PROGRAM**

(71) Applicant: Fujitsu Limited, Kawasaki-shi, Kanagawa 211-8588 (JP)
(72) Inventor: MI, Xiaoyu, Kawasaki-shi Kanagawa 211-8588 (JP); UEDA, Satoshi, Kawasaki-shi Kanagawa 211-8588 (JP); TOYODA, Osamu, Kawasaki-shi Kanagawa 211-8588 (JP); NAKAZAWA, Fumihiko, Kawasaki-shi Kanagawa 211-8588 (JP)
(74) Representative: Ward, James Norman
(86) International application number: PCT/JP2013/063174
(87) International publication number: WO 2014/181467

(57) **Abstract**

A sensor part has a plurality of sensor cells provided in a matrix arrangement on a sensor that detects a pulse within a region in which the pulse of a diagnostic target is detected, in a state in which a pressure is applied to the region through the sensor. A diagnostic apparatus acquires a distribution of a pulse waveform based on the pulse detected in a state in which the pressure is constant, determines a plurality of observation positions within the region, and determines a maximum amplitude at the plurality of observation positions that are determined by increasing the pressure, based on the distribution of the pulse waveform. A digitized score of the pulse at each observation position is computed based on the pressure at a time when the pulse waveform having the maximum amplitude is obtained at each observation position, and scores at the plurality of observation positions are integrated. A reference is made to a database prestoring analogized states of the diagnostic target with respect to the scores, based on the integrated score, in order to analogize the state (pattern) of the diagnostic target, and generate a diagnostic result to be output with respect to the diagnostic target from the analogized state (pattern) of the diagnostic target.

## Description

### TECHNICAL FIELD

The present invention relates to a diagnostic apparatus, a diagnostic method, and a program.

### BACKGROUND ART

A diagnostic method of oriental medicine, including Chinese herbal medicine, includes 4 methods of examination (four examinations) called inspection, listening and smelling examination, inquiry, and palpation (or touch). The inspection makes an eye-observation of the patient's physique, complexion, tongue, or the like, in order to make a diagnosis related to body temperature, body moisture, abnormality in blood circulation state, progression of illness, or the like. The listening and smelling examination makes an ear-observation of the patient's voice, cough, breathing sound, borborygmus or the like, or makes a nose-observation of patient's body odor, halitosis, odor of urine, feces and secretion, or the like, in order to make a diagnosis related to patient's illness. The inquiry makes inquires to acquire the patient's little symptoms such as hot sensation, chill, stiffness in shoulder, pain, or the like, in order to make a diagnosis. The palpation makes a hand-observation by a practitioner to examine properties or shapes of the patient's pulse (so-called pulse examination), or to examine epigastric tension or stimulated reaction to direct touch to the stomach (so-called abdominal examination), in order to make a diagnosis. Amongst the four examinations, one characteristic of the palpation is that the practitioner senses the patient's biometric information by direct touch by hand.

The diagnostic method of the oriental medicine is a subjective method that makes the examination based on patient's information acquired by five senses of the practitioner, and prescribes appropriate Chinese herbal medicine according to the patient's age, physique, or the like. For this reason, the practitioner is required to have extensive knowledge, expert skills, or the like. On the other hand, because examination procedures carried out by the practitioner differ for each practitioner, and diagnostic results may differ for each practitioner and lack objectivity. Particularly in the case of the palpation in which the practitioner senses the patient's biometric information by direct touch by hand, the diagnostic results of the palpation depend greatly on the practitioner.

For example, in the case of the pulse examination, the practitioner needs to accurately determine (or identify) positions of the patient's body parts called inch, bar, and cubit based on experience or the like, in order to sense the pulse by touch. Further, in addition to the pulse rate, the practitioner needs to accurately judge, based on experience or the like, pulse signs including the depth of pulse (floating pulse to sunken pulse), amplitude of pulse (deficient pulse to excessive pulse), period of pulse (rapid pulse to slow pulse), length and width of pulse (large pulse to small pulse), fluency of pulse (smooth pulse to rough pulse), tonus of pulse (tension pulse to relaxed pulse), or the like at the inch, bar, and cubit of the patient's hand. For this reason, the diagnostic results of the palpation depend on the accuracy of the positions of the patient's inch, bar, and cubit determined by the practitioner, the accuracy of the pulse signs judged by directly touching the patient's inch, bar, and cubit by the practitioner's index finger, middle finger, and ring finger, or the like. In other words, the diagnostic results of the palpation based on the five senses of the practitioner greatly depend on the practitioner who makes the examination.

### PRIOR ART DOCUMENTS

### PATENT DOCUMENTS

Patent Document 1: Japanese Laid-Open Patent Publication No. 11-19055
Patent Document 2: Japanese Laid-Open Patent Publication No. 6-197873
Patent Document 3: Japanese Laid-Open Patent Publication No. 6-254060
Patent Document 4: Japanese Laid-Open Patent Publication No. 2004-208711

### DISCLOSURE OF THE INVENTION

### PROBLEM TO BE SOLVED BY THE INVENTION

According to the conventional diagnostic method that performs the palpation based on the pulse, the diagnostic results greatly depend on the practitioner.

Hence, one object of the present invention is to provide a diagnostic apparatus, a diagnostic method, and a program which can perform the palpation based on the pulse, without being greatly dependent on the practitioner.

### MEANS OF SOLVING THE PROBLEM

According to one aspect of the present invention, there is provided a diagnostic apparatus including a sensor part having a plurality of sensor cells provided in a matrix arrangement on a sensor that detects a pulse within a region in which the pulse of a diagnostic target is detected, in a state in which a pressure is applied to the region through the sensor; means for acquiring a distribution of a pulse waveform, based on a sensor signal indicating the pulse detected by the sensor part in a state in which the pressure is constant; means for determining a plurality of observation positions within the region, and determining a pulse waveform having a maximum amplitude at the plurality of observation positions that are determined by increasing the pressure, based on the distribution of the pulse waveform; means for computing a digitized score of the pulse at each observation position, based on the pressure at a time when the pulse waveform having the maximum amplitude is obtained at each observation position; means for analogizing a state of the diagnosis target, by integrating scores at the plurality of observation positions, and referring to a database that prestores analogized states of the diagnosis target with respect to the scores, based on an integrated score; and means for generating a diagnostic result with respect to the diagnostic target from the analogized state of the diagnostic target, and outputting the diagnostic result.

### EFFECTS OF THE INVENTION

According to the disclosed diagnostic apparatus, diagnostic method, and program, the palpation based on the pulse can be performed without being greatly dependent on the practitioner.

### BRIEF DESCRIPTION OF THE DRAWING

FIG. 1 is a block diagram illustrating an example of a computer system;
FIG. 2 is a diagram for explaining positions of inch, bar, and cubit of a patient's hand;
FIG. 3 is a diagram illustrating an example of a pulse waveform at a certain observation position;
FIG. 4 is a plan view illustrating an example of a sensor of a sensor part 11;
FIG. 5 is a diagram for explaining states of the sensor and the patient's hand;
FIG. 6 is a schematic diagram illustrating wirings of the sensor;
FIG. 7 is a cross sectional view illustrating a first example of a configuration of a sensor cell;
FIG. 8 is a cross sectional view illustrating a second example of the configuration of the sensor cell;
FIG. 9 is a cross sectional view illustrating a third example of the configuration of the sensor cell;
FIG. 10 is a cross sectional view illustrating a fourth example of the configuration of the sensor cell;
FIG. 11 is a flow chart for explaining an example of a diagnostic process;
FIG. 12 is a flow chart for explaining an example of an inch, bar, and cubit determination process;
FIG. 13 is a diagram illustrating an amplitude of an acquired pulse waveform along an X-axis direction;
FIG. 14 is a diagram illustrating an amplitude of the acquired pulse waveform along a Y-axis direction;
FIG. 15 is a flow chart for explaining an example of a bar's optimum pulse waveform determination process;
FIG. 16 is a diagram illustrating an example of a relationship between the amplitude of the pulse waveform and a pressure applied at the observation positions;
FIG. 17 is a diagram illustrating an example of a relationship between a pulse response and time for a case in which different pressures are applied at the observation position;
FIG. 18 is a flow chart for explaining an example of an inch's optimum pulse waveform determination process;
FIG. 19 is a flow chart for explaining an example of a cubit's optimum pulse waveform determination process;
FIG. 20 is a flow chart for explaining an example of a bar, inch and cubit's optimum pulse waveform determination process for a case in which pressure is simultaneously applied to the bar, inch, and cubit;
FIG. 21 is a diagram for explaining an example of a learning process of a database;
FIG. 22 is a diagram illustrating an example of the sensor part attached to the patient's hand; and
FIG. 23 is a diagram for explaining the configuration of the sensor part illustrated in FIG. 22.

### MODE OF CARRYING OUT THE INVENTION

According to the disclosed diagnostic apparatus, diagnostic method, and program uses a sensor part having a plurality of sensor cells provided in a matrix arrangement on a sensor that detects a pulse within a region in which the pulse of a diagnostic target is detected, in a state in which a pressure is applied to the region through the sensor. A distribution of a pulse waveform is acquired based on the pulse detected in a state in which the pressure is constant, a plurality of observation positions within the region are determined, and a maximum amplitude at the plurality of observation positions that are determined by increasing the pressure are determined, based on the distribution of the pulse waveform. A digitized score of the pulse at each observation position is computed based on the pressure at a time when the pulse waveform having the maximum amplitude is obtained at each observation position, and scores at the plurality of observation positions are integrated. A reference is made to a database prestoring analogized states of the diagnostic target with respect to the scores, based on the integrated score, in order to analogize the state of the diagnostic target, and generate a diagnostic result to be output with respect to the diagnostic target from the analogized state of the diagnostic target.

Next, a description will be given of each embodiment of the disclosed diagnostic apparatus, diagnostic method, and program, by referring to the drawings.

### EMBODIMENTS

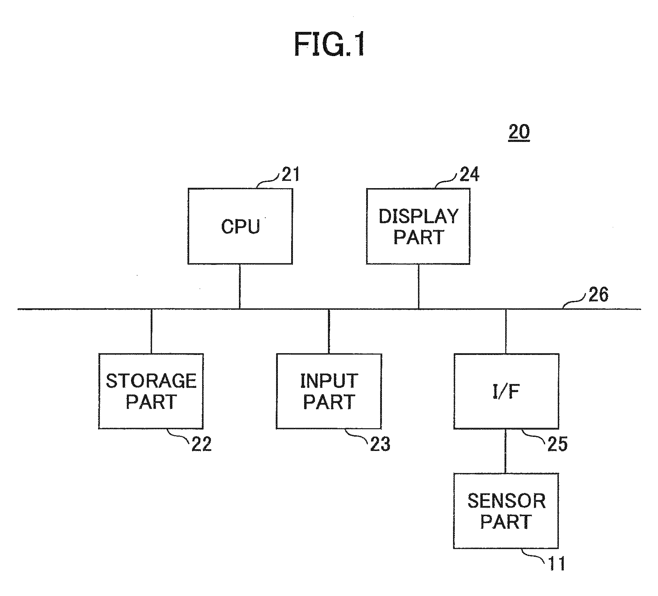

FIG. 1 is a block diagram illustrating an example of a computer system. A computer system 20 illustrated in FIG. 1 has a configuration in which a CPU (Central Processing Unit) 21 that is an example of a processor, a storage part 22, an input part 23, a display part 24, and an interface (I/F) 25 are connected via a bus 26. Of course, the connection of each of the parts within the computer system 20 is not limited to the connection using the bus 26 illustrated in FIG. 1. In addition, a DSP (Digital Signal Processor) may be used in place of the CPU 21.

The CPU 21 controls the entire computer system 20, and can perform a diagnostic process which will be described later by executing a program, and can perform functions of a diagnostic apparatus together with a sensor part 11. The storage part 22 stores the program to be executed by the CPU 21, intermediate results of computations executed by the CPU 21, data used by the program and the computations executed by the CPU 21, a database, or the like. As will be described later, the database stores, with respect to scores of element parameters of the pulse, analogical results or the like on a patient who is a diagnostic target, such as the patient's abnormal solid and hollow viscera (internal organ), human constitution, pathological condition (pattern), illness, therapeutic effect, or the like according to known theories of oriental medicine. The storage part 22 may be formed by a computer-readable storage medium. The computer-readable storage medium may be a semiconductor memory device. In addition, in a case in which the computer-readable storage medium is a magnetic recording medium, an optical recording medium, a magneto-optic recording medium, or the like, the storage part 22 may be formed by a reader and writer that reads and writes information with respect to the recording medium that is loaded into the reader and writer. The input part 23 may be formed by a keyboard or the like, and is used to input commands, data, or the like to the computer system 20. The display part 24 displays messages to an operator, data related to the diagnostic process, or the like. The I/F 25 is communicable with an external device, including the sensor part 11, by cable or wireless communication.

The diagnostic apparatus in one embodiment may be formed by the computer system 20 and the sensor part 11.

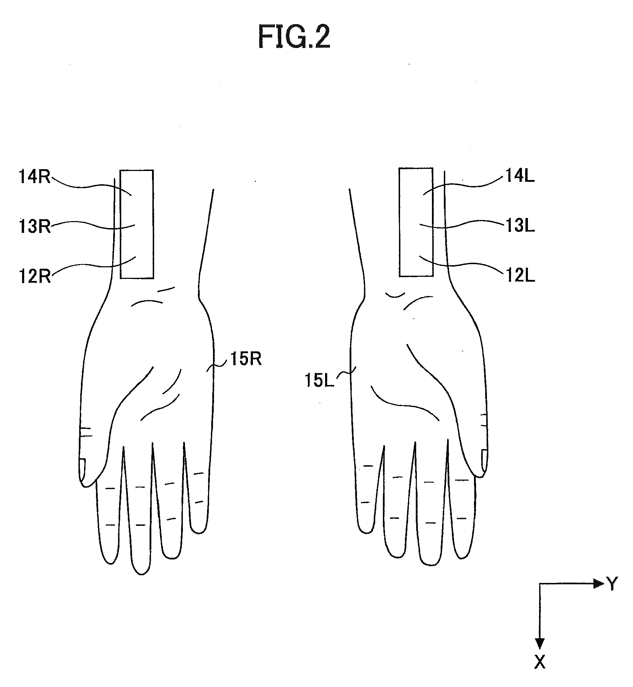

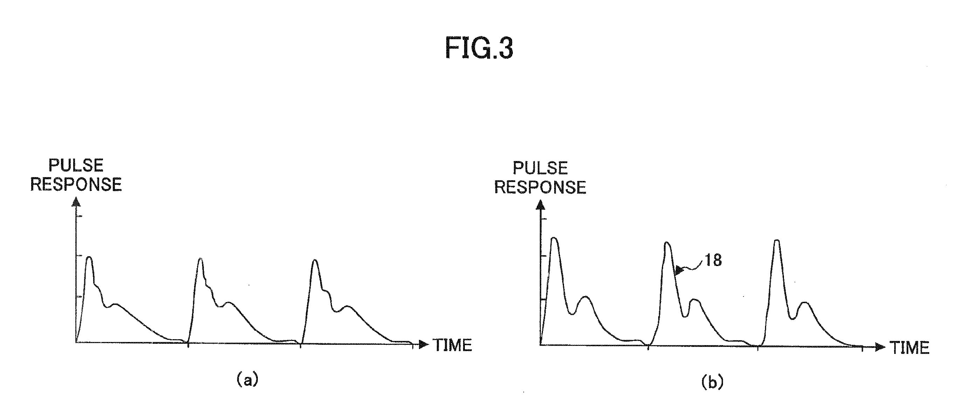

FIG. 2 is a diagram for explaining positions of inch, bar, and cubit of a patient's hand. In FIG. 2, the inch, bar, and cubit of a patient's right hand 15R are located at positions indicated by 12R, 13R, and 14R. In addition, the inch, bar, and cubit of a patient's left hand 15L are located at positions indicated by 12L, 13L, and 14L. For example, in the case of the pulse examination, it is possible to analogize and diagnose the patient's abnormal solid and hollow viscera (internal organ), human constitution, pathological condition (pattern), illness, therapeutic effect, or the like according to known theories of oriental medicine, based on pulse signs including the pulse rate, the depth of pulse (floating pulse to sunken pulse), amplitude of pulse (deficient pulse to excessive pulse), period of pulse (rapid pulse to slow pulse), length and width of pulse (large pulse to small pulse), fluency of pulse (smooth pulse to rough pulse), tonus of pulse (tension pulse to relaxed pulse), or the like at observation positions called the patient's inches 12R and 12L, bars 13R and 13L, and cubits 14R and 14L, that is, based on 6 pairs of element parameters of the pulse. FIG. 3 is a diagram illustrating an example of a pulse waveform at a certain observation position. In FIG. 3, the ordinate indicates a pulse response in arbitrary units, and the abscissa indicates the time in arbitrary units. In FIG. 3, (a) illustrates the pulse waveform of the patient in a healthy state, and (b) illustrates the pulse waveform of the same patient in a state in which the health is deteriorated. It is possible to analogize and diagnose, from the state of a smooth pulse 18 in (b) of FIG. 3, the patient's fluid retention, food retention, excess and heat, or the like, for example, according to known theories of oriental medicine.

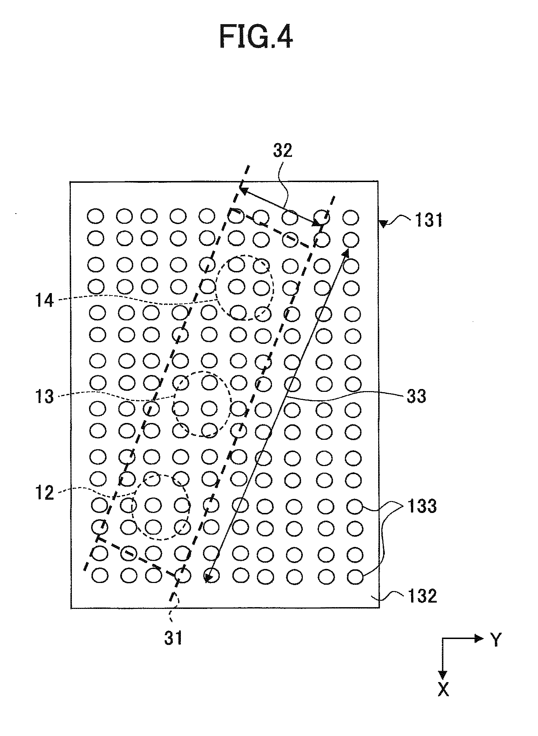

FIG. 4 is a plan view illustrating an example of a sensor of a sensor part 11. A sensor (or sensor sheet) 131 includes a sheet 132, and a plurality of sensor cells 133 provided in a 2-dimensional matrix arrangement on the sheet 132. The sensor cell 133 has an area sufficient to detect the inch, bar, and cubit parts when the sheet 132 is attached to the patient's hand, that is, has an area in which the pressure is detectable within a predetermined region of the diagnostic target. The sensor cell 133 can detect an xy-coordinate position on the sheet 132 where the pressure is applied, and a change in the pressure with respect to time. Accordingly, it is possible to detect distributions of pulse waveforms based on detection signals from the plurality of sensor cells 133. In FIG. 4, a coarse dotted line 31 indicates an estimated range of arteria radialis of the patient's hand positioned under the sensor 13, and circular marks 12, 13, and 14 in fine dotted lines indicate estimated ranges of the inch, bar, and cubit of the patient's hand positioned under the sensor 131, respectively. The estimated range 31 of the arteria radialis and the estimated ranges 12, 13, and 14 of the inch, bar, and cubit may be set to an average range of the human arteria radialis and average ranges of the inch, bar, and cubit, respectively, or to ranges added with predetermined error margins to the average ranges, respectively. In addition, a reference numeral 32 indicates an estimated width of the pulse, and a reference numeral 33 indicates an estimated length of the pulse. The size of the arteria radialis 31 at the time of beating may have individual differences, however, the estimated length of the pulse is approximately 3 mm, for example. As will be described later, this embodiment determines the positions of the inch, bar, and cubit of the patient and the optimum pulse waveform, based on sensor information from the sensor 131. In addition, it is also possible to determine the width and the length of the pulse based on the sensor information.

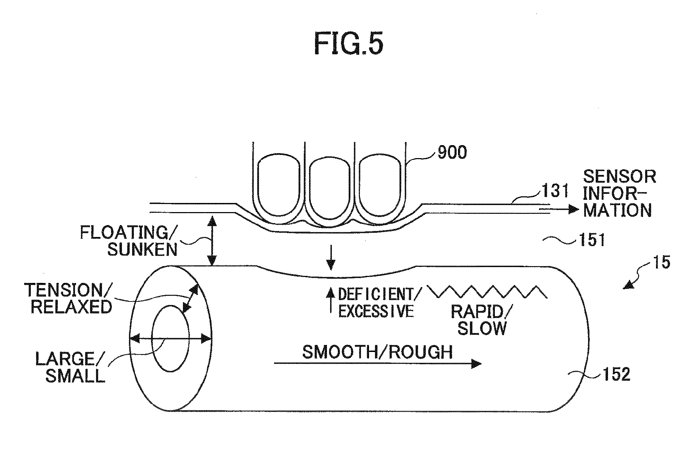

FIG. 5 is a diagram for explaining states of the sensor and the patient's hand. In FIG. 5, the sensor 131 is attached to a skin 151 of a patient's hand 15, so as to cover the parts of the inch 12, the bar 13, and the cubit 14 of the patient's hand 15. A blood vessel 151 that is a measuring target is located under the skin 151. In this example, a practitioner 900 pushes the sensor 131 in a direction of the skin 151 by the practitioner's finger tips, so as to apply pressure to the sensor 131 and the blood vessel 152, however, the sensor 131 may mechanically apply the pressure, as will be described later. The sensor 131 detects the pulse in the blood vessel 152, and outputs analog sensor information (that is, an analog sensor output signal). The analog sensor output signal may be converted into a digital signal by an analog-to-digital converter (ADC, not illustrated) within the sensor part 11 before being output to the computer system 20, or may be output to the computer system 20 through an external signal processing circuit or the like that includes an ADC.

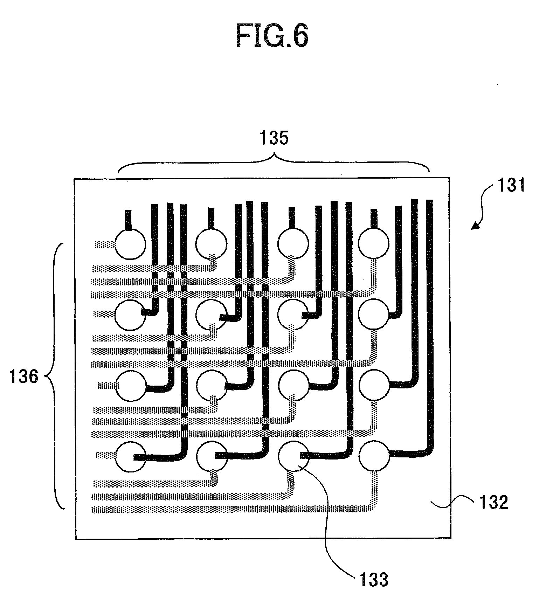

FIG. 6 is a schematic diagram illustrating wirings of the sensor. In FIG. 6, upper layer wirings 135 of the sensor 131 are indicated in black color, and lower layer wirings 136 are indicated in gray color. The shape of the sensor cells 131 may be other than the circular shape.

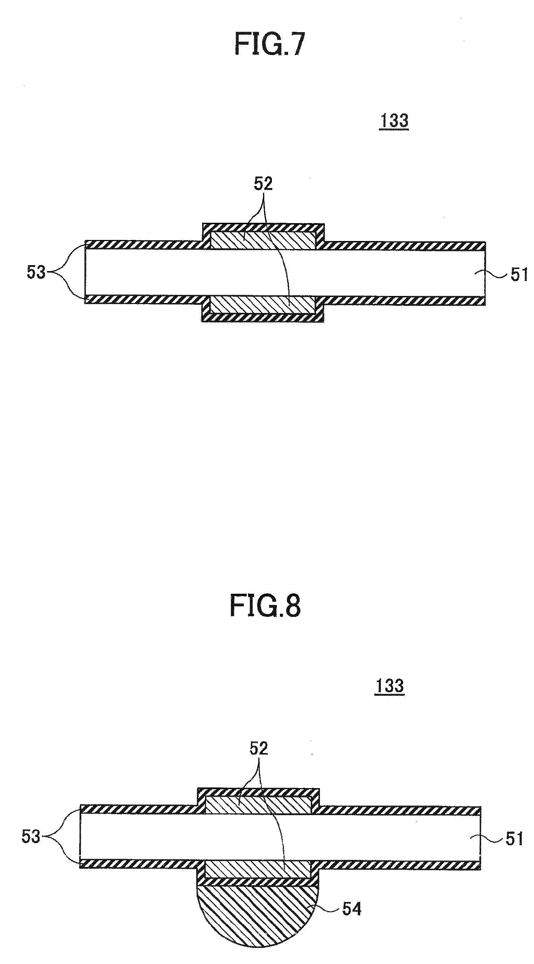

FIG. 7 is a cross sectional view illustrating a first example of a configuration of the sensor cell. In FIG. 7, the sensor cell 133 has the configuration in which a metal electrode film 52 is formed on upper and lower surfaces of a sensing thin film 51 that is formed by a sensing material such as an organic electret material or the like, and the entire sensor cell 133 is covered by an insulator thin film 53. The sensing material preferably has a high sensitivity with respect to positive pressure, and a sensitivity close to zero with respect to shear force. For example, in a case in which a piezoelectric material or an organic electret material is used for the sensing material, g33 and d33 are preferably 10 times or more compared to g31 and d31. The sensor 131 is preferably formed by a thin and flexible (that is, soft) sheet, so as to sufficiently conform to movements of tissues (that is, skin 151 and blood vessel 152) of the patient's hand 15 in FIG. 5, for example. In addition, from a viewpoint of facilitating transfer of the pulse to the hand of the practitioner 900 through the sensor 131 in FIG. 5, for example, the sensor 131 is preferably formed by a thin and flexible sheet. Young's modulus of the sensing thin film 51 is desirably 1 GPa or lower, for example, and the film thickness of the sensing thin film 51 is preferably 100 µm or less, for example.

FIG. 8 is a cross sectional view illustrating a second example of the configuration of the sensor cell. In FIG. 8, those parts that are the same as those corresponding parts in FIG. 7 are designated by the same reference numerals, and a description thereof will be omitted. The sensor cell 133 illustrated in FIG. 8 has a projection 54 formed on the metal electrode film 52 on one side thereof, via the insulator thin film 53. When attaching the sensor 131 to the patient's hand 15, the projection 54 may face the patient's hand 15, or may face the hand of the practitioner. By providing the projection 54, it is possible to improve the sensitivity of the sensor cell 133.

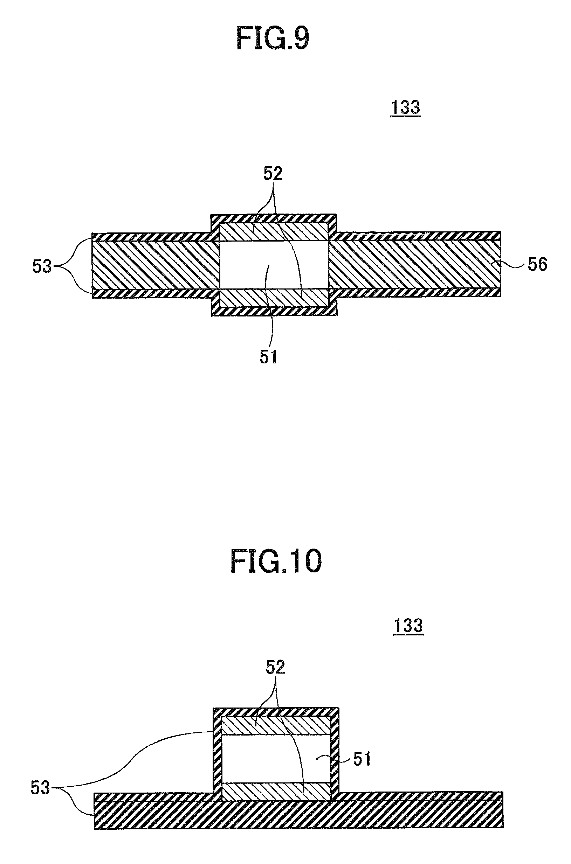

FIG. 9 is a cross sectional view illustrating a third example of the configuration of the sensor cell. In FIG. 9, those parts that are the same as those corresponding parts in FIG. 7 are designated by the same reference numerals, and a description thereof will be omitted. In the sensor cell 133 illustrated in FIG. 9, a sensor region sandwiched between metal electrode films 52 is formed by the sensing thin film 51, and regions 56 other than the sensor region are formed by a material having a pressure sensitivity lower than that of the sensor region, or by a material having no pressure sensitivity.

FIG. 10 is a cross sectional view illustrating a fourth example of the configuration of the sensor cell. In FIG. 10, those parts that are the same as those corresponding parts in FIG. 7 are designated by the same reference numerals, and a description thereof will be omitted. In the sensor cell 133 illustrated in FIG. 10, only a sensor region sandwiched between the metal electrode films 52 is formed by the sensing thin film 51. For example, the configuration illustrated in FIG. 10 may be formed by removing the sensing thin film 51 in regions other than the sensor region, or by locally printing the sensor region by a printing method.

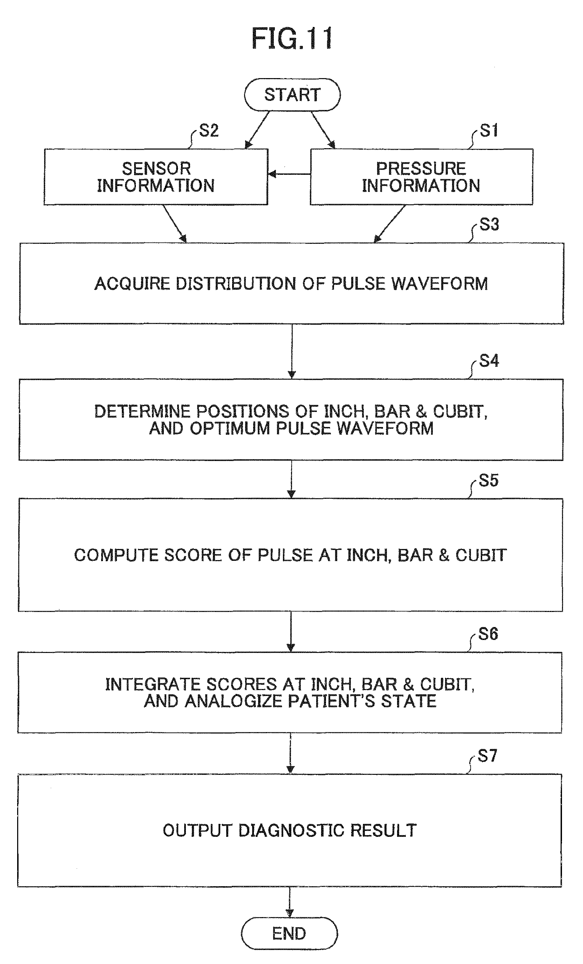

FIG. 11 is a flow chart for explaining an example of a diagnostic process. In FIG. 11, step S2 may be executed by the sensor part 11, and steps S1 and S3 through S7 may be executed by the CPU 21, for example. It is assumed that sensors 131 having the same configuration are attached to the patient's right hand and left hand.

In FIG. 11, in step S1, pressure information (or pressure signal) indicating a pressure to be applied to the patient's hand 15 through the sensor 131 is input to the computer system 20. In the case in which the practitioner applies the pressure by the practitioner's hand, the pressure information may be input from the input part 23, for example, or it is possible to input a default value stored in the storage part 22. In addition, in the case in which the sensor part 11 mechanically applies the pressure as will be described later, it is possible to input the pressure information that is stored in the storage part 22 and is to be supplied to a pressure applying mechanism of the sensor part 11, for example. Further, the pressure information may be detected by the sensor 131. It is assumed that the pressure is constant when determining the positions of the patient's inch, bar, and cubit, and that the pressure is gradually increased when determining the optimum pulse waveform. In step S2, the sensor information (or sensor output signal) from the sensor 131 is input to the computer system 20.

In step S3, the distributions of the pulse waveforms are acquired based on the sensor information. In step S4, 6 observation positions, that is, the position of the inch, the position of the bar, and the position of the cubit of the patient's right hand, and the position of the inch, the position of the bar, and the position of the cubit of the patient's left hand, are determined based on the distributions of the pulse waveforms, and the optimum pulse waveforms at these 6 determined observation positions are determined.

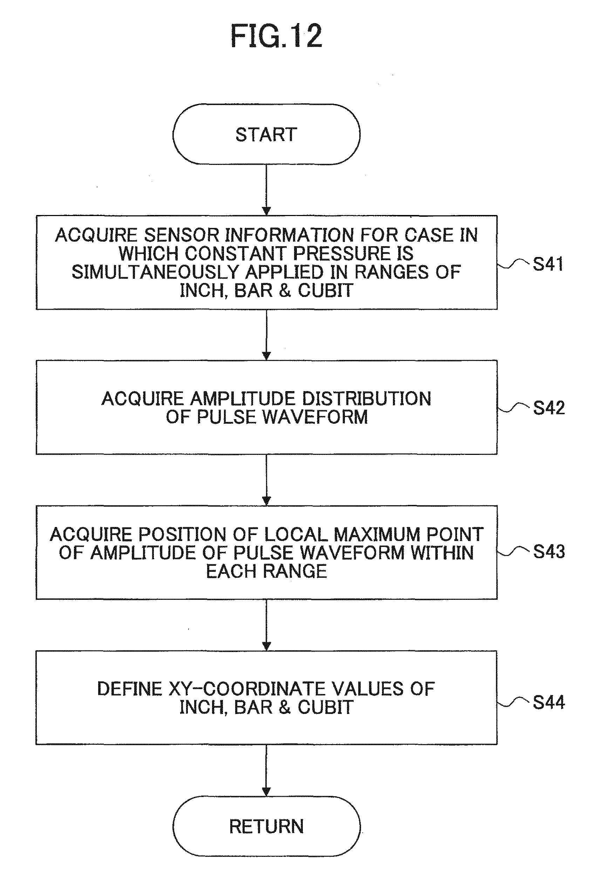

FIG. 12 is a flow chart for explaining an example of an inch, bar, and cubit determination process of step S4. In step S41 illustrated in FIG. 12, the sensor information is acquired for a case in which a constant pressure is simultaneously applied to the estimated ranges 12, 13, and 14 of the inch, bar, and cubit illustrated in FIG. 4. In step S42, the amplitude distributions of the pulse waveforms within the estimated ranges 12, 13, and 14 of the inch, bar, and cubit are acquired, based on the acquired sensor information. In step S43, the positions of local maximum points of the amplitudes of the pulse waveforms within the estimated ranges 12, 13, and 14 of the inch, bar, and cubit are acquired.

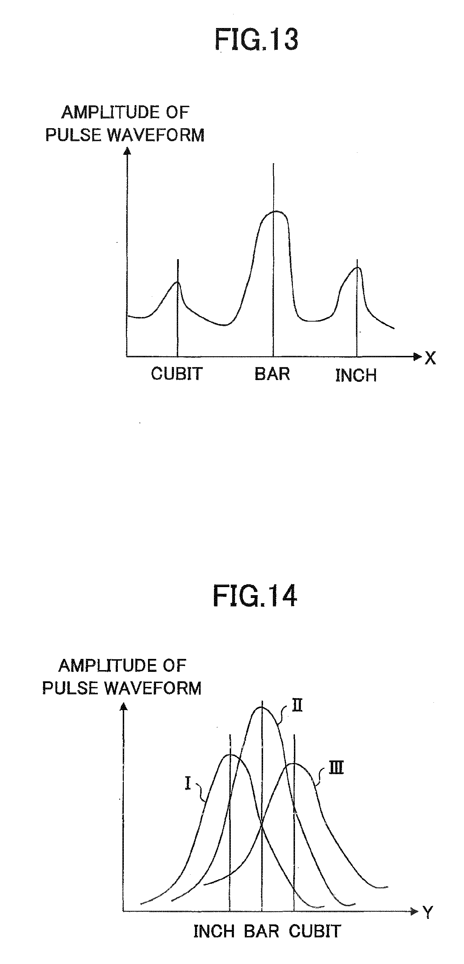

FIG. 13 is a diagram illustrating the amplitude of the acquired pulse waveform along an X-axis direction, and the ordinate indicates the amplitude of the pulse waveform in arbitrary units. FIG. 14 is a diagram illustrating the amplitude of the acquired pulse waveform along a Y-axis direction, and the ordinate indicates the amplitude of the pulse waveform in arbitrary units. In FIG. 13, 3 peaks in the amplitude of the pulse waveform correspond to the X-coordinate positions of the inch, bar, and cubit, respectively. Similarly, in FIG. 14, 3 peaks in the amplitudes of the pulse waveforms correspond to the Y-coordinate positions of the inch, bar, and cubit, respectively. In FIG. 14, I indicates the distribution of the amplitude of the pulse waveform in the estimated range 12 of the inch along the Y-axis, II indicates the distribution of the amplitude of the pulse waveform in the estimated range 13 of the bar along the Y-axis, and III indicates the distribution of the amplitude of the pulse waveform in the estimated range 14 of the cubit along the Y-axis.

In step S44, the X-coordinate of the peak on the right side of FIG. 13 and the Y-coordinate of the peak on the left side in FIG. 14 are defined as the XY-coordinate values of the inch, the X-coordinate of the peak at the center in FIG. 13 and the Y-coordinate of the peak at the center in FIG. 14 are defined as the XY-coordinate values of the bar, and the X-coordinate of the peak on the left side of FIG. 13 and the Y-coordinate of the peak on the right side in FIG. 14 are defined as the XY-coordinate values of the cubit, and the process returns to step S4 illustrated in FIG. 11.

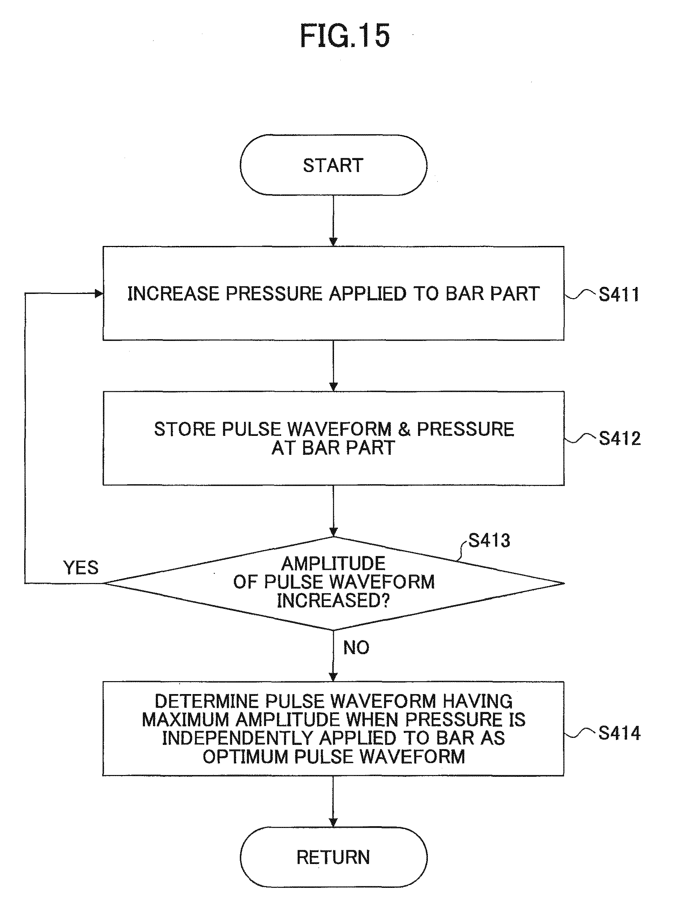

FIG. 15 is a flow chart for explaining an example of a bar's optimum pulse waveform determination process of step S4. In step S411 illustrated in FIG. 15, the pressure applied to the determined XY-coordinate position of the bar is increased by a predetermined amount. In step S412, the pulse waveform at the bar and the pressure applied to the bar at this point in time are stored in the storage part 22. In step S413, a judgement is made to determine whether the amplitude of the pulse waveform increased, and the process returns to step S411 when the judgment result is YES, and the process advances to step S414 when the judgment result is NO. In step S414, the pulse waveform having the maximum amplitude amongst the pulse waveforms stored in the storage part 22 is determined as the optimum pulse waveform for the case in which the pressure is independently applied to the bar.

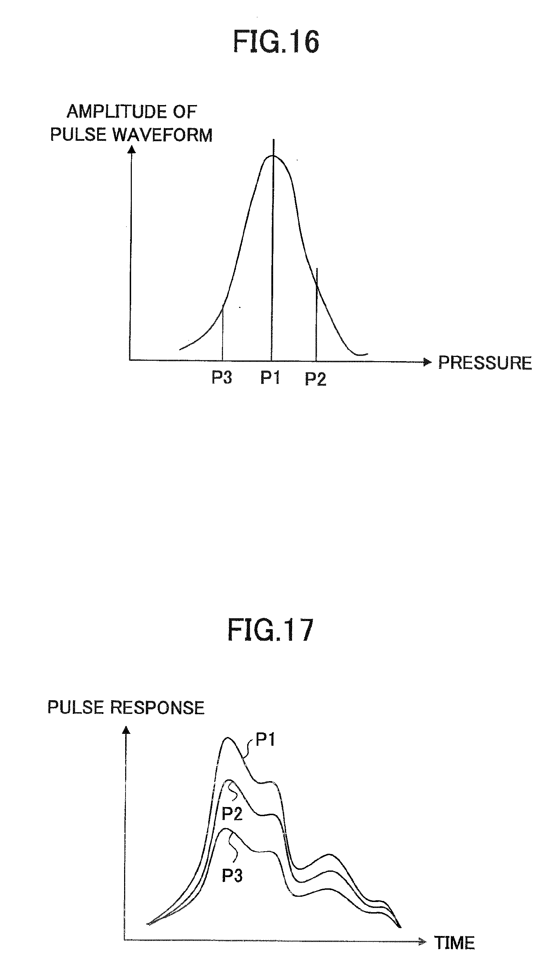

FIG. 16 is a diagram illustrating an example of a relationship between the amplitude of the pulse waveform and the pressure applied at the observation positions such as the inch, bar, and cubit. In FIG. 16, the ordinate indicates the amplitude of the pulse waveform in arbitrary units, and the abscissa indicates the pressure applied at the observation positions such as the inch, bar, and cubit. In the example illustrated in FIG. 16, the pulse waveform when the pressure is P1 has the maximum amplitude, where P3 < P1 < P2.

FIG. 17 is a diagram illustrating an example of a relationship between a pulse response and time for a case in which different pressures are applied at the observation position such as the inch, bar, and cubit. In FIG. 17, the ordinate indicates the pulse response in arbitrary units, and the abscissa indicates the time in arbitrary units.

In the case of the examples illustrated in FIGs. 16 and 17, when the observation position is assumed to be the bar, the pulse waveform when the pressure is P1 in FIG. 17 is determined as the optimum pulse waveform at the bar in step S414.

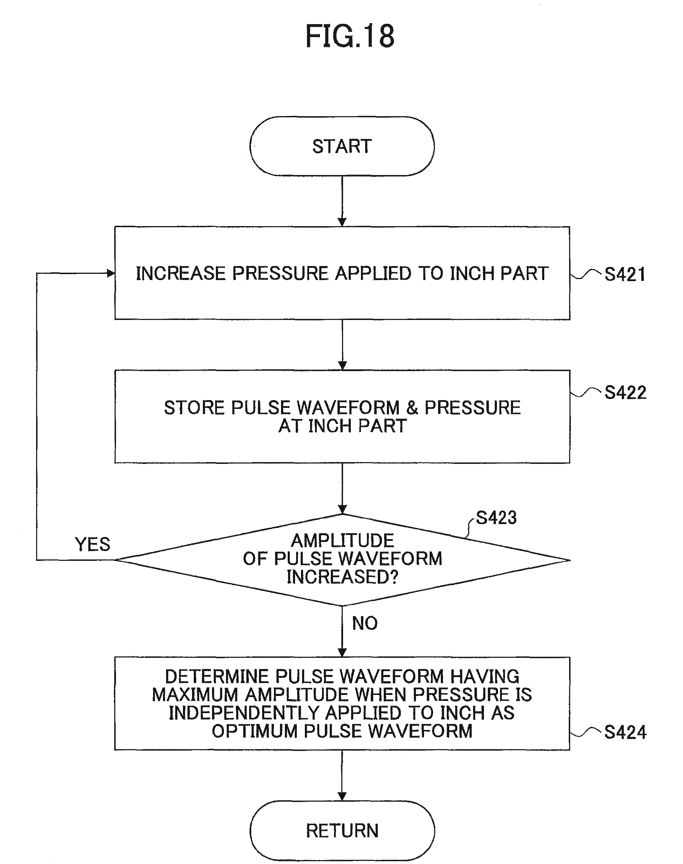

FIG. 18 is a flow chart for explaining an example of an inch's optimum pulse waveform determination process of step S4. In step S421 illustrated in FIG. 18, the pressure applied to the determined XY-coordinate position of the inch is increased by a predetermined amount. In step S422, the pulse waveform at the inch and the pressure applied to the inch at this point in time are stored in the storage part 22. In step S423, a judgement is made to determine whether the amplitude of the pulse waveform increased, and the process returns to step S421 when the judgment result is YES, and the process advances to step S424 when the judgment result is NO. In step S424, the pulse waveform having the maximum amplitude amongst the pulse waveforms stored in the storage part 22 is determined as the optimum pulse waveform for the case in which the pressure is independently applied to the inch.

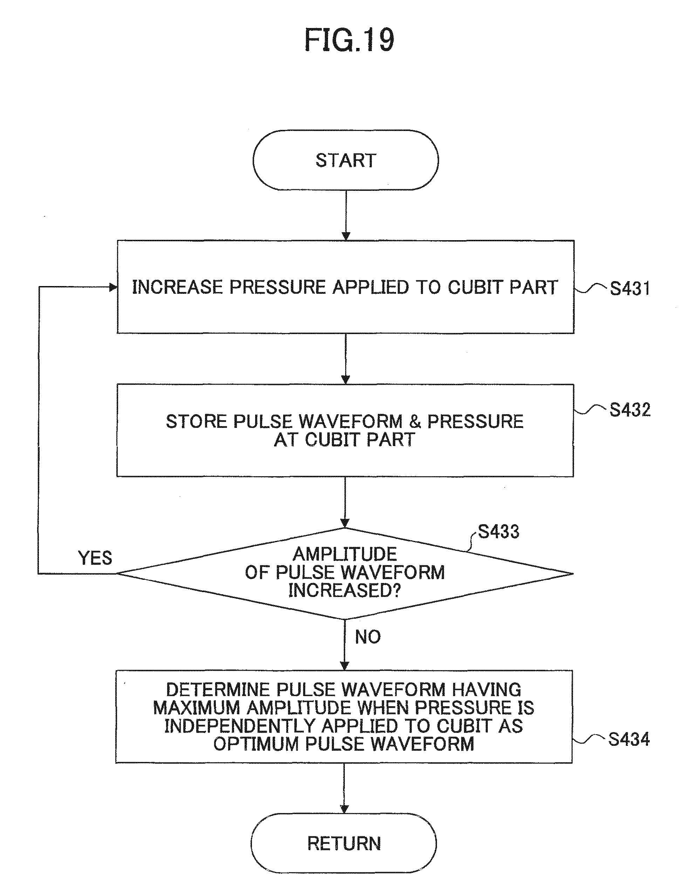

FIG. 19 is a flow chart for explaining an example of a cubit's optimum pulse waveform determination process of step S4. In step S431 illustrated in FIG. 19, the pressure applied to the determined XY-coordinate position of the cubit is increased by a predetermined amount. In step S432, the pulse waveform at the cubit and the pressure applied to the cubit at this point in time are stored in the storage part 22. In step S433, a judgement is made to determine whether the amplitude of the pulse waveform increased, and the process returns to step S431 when the judgment result is YES, and the process advances to step S434 when the judgment result is NO. In step S434, the pulse waveform having the maximum amplitude amongst the pulse waveforms stored in the storage part 22 is determined as the optimum pulse waveform for the case in which the pressure is independently applied to the cubit.

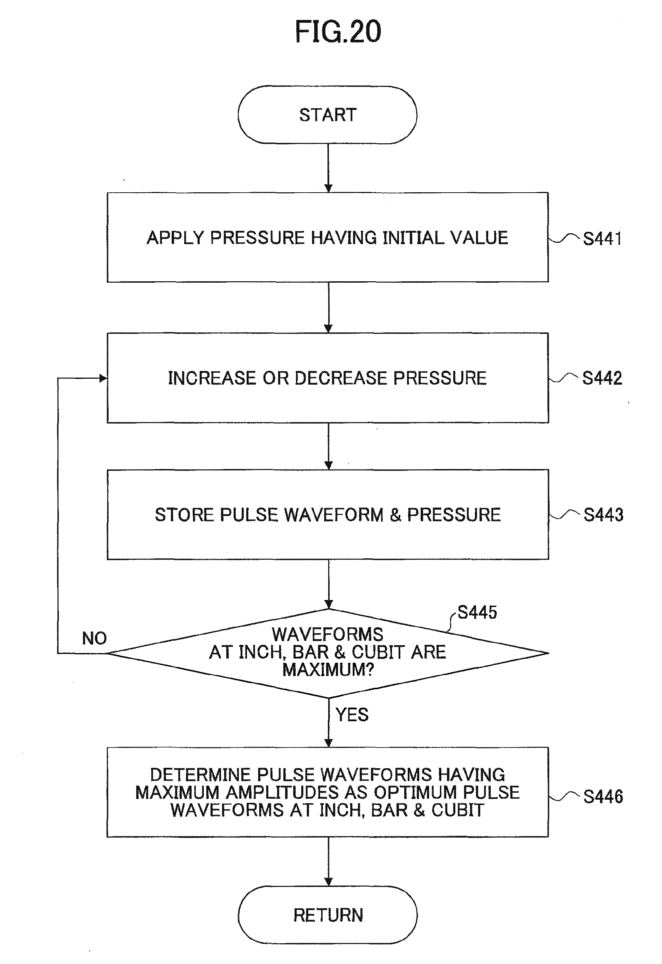

FIG. 20 is a flow chart for explaining an example of a bar, inch and cubit's optimum pulse waveform determination process of step S4 for a case in which pressure is simultaneously applied to the bar, inch, and cubit. In step S441 illustrated in FIG. 20, the pressure is applied at each of the observation positions of the bar, inch, and cubit, using, as initial values, the pressures at which the maximum amplitudes are obtained when the pressures are independently applied to the bar, inch, and cubit as described above. In step S442, the pressure applied at each of the observation positions of the bar, inch, and cubit is increased or decreased by a predetermined amount. In step S443, the pulse waveforms at the observation positions of the bar, inch, and cubit, and the pressures applied at the observation positions of the bar, inch, and cubit at this point in time are stored in the storage part 22. In step S445, a judgment is made to determine whether each of the pulse waveforms at the observation positions of the bar, inch, and cubit has the maximum amplitude (amplitude greater than or equal to the maximum amplitudes at the time when the pressures are independently applied to the bar, inch, and cubit). The process returns to step S442 when the judgment result in step S445 is NO, and the process advances to step S446 when the judgment result in step S445 is YES. In step S446, the pulse waveforms having the maximum amplitude at the observation positions of the bar, inch, and cubit, respectively, amongst the pulse waveforms stored in the storage part 22, are determined as the optimum pulse waveforms for the case in which the pressure is simultaneously applied to the bar, inch, and cubit.

Returning now to the description of FIG. 11, in step S5, a core is computed with respect to the pulse at the total of 6 observation positions that are the inches, bars, and cubits of the patient's right and left hands. In other words, the pulse at each observation position is digitized according to a predetermined algorithm based on the pressure that is applied to each observation position at the time when the optimum pulse waveform is obtained. More particularly, the score is computed according to the predetermined algorithm with respect to the pulse signs (element parameters) including the depth of pulse (floating pulse to sunken pulse), amplitude of pulse (deficient pulse to excessive pulse), period of pulse (rapid pulse to slow pulse), length and width of pulse (large pulse to small pulse), fluency of pulse (smooth pulse to rough pulse), tonus of pulse (tension pulse to relaxed pulse), or the like. For example, a table recorded with the scores with respect to the pressures that are applied when the optimum pulse waveforms are obtained at the observation positions may be stored in the storage part 22. In this case, in step S5, the scores may be obtained with respect to the pulses at the total of 6 observation points by referring to this table.

In step S6, the scores at the total of 6 observation points are integrated, and the patient's state may be analogized by making a reference to the database based on the integrated score. The database stores the analogized results or the like, such as the patient's abnormal solid and hollow viscera (internal organ), human constitution, pathological condition (pattern), illness, therapeutic effect, or the like according to known theories of oriental medicine, with respect to the scores of the element parameters of the pulses. The database preferably stores at least one analogized result amongst the patient's abnormal solid and hollow viscera (internal organ), human constitution, pathological condition (pattern), illness, therapeutic effect, or the like, with respect to the scores of the element parameters of the pulses. The database more preferably stores two or more analogized results amongst the patient's abnormal solid and hollow viscera (internal organ), human constitution, pathological condition (pattern), illness, therapeutic effect, or the like, with respect to the scores of the element parameters of the pulses. The database may be stored in the storage part 22, for example, or may be stored in an external storage part (not illustrated). In step S7, a diagnostic result is generated from the analogized result obtained in step S6, this diagnostic result is output, and the diagnostic process ends. The diagnostic result may include the score, the patient's abnormal solid and hollow viscera (internal organ), human constitution, pathological condition (pattern), therapeutic effect, or the like. For example, the diagnostic result may be output to and displayed on the display part 24, stored in the storage part 22 as a part of an electronic medical record, or output to an external device (not illustrated) via the I/F 25.

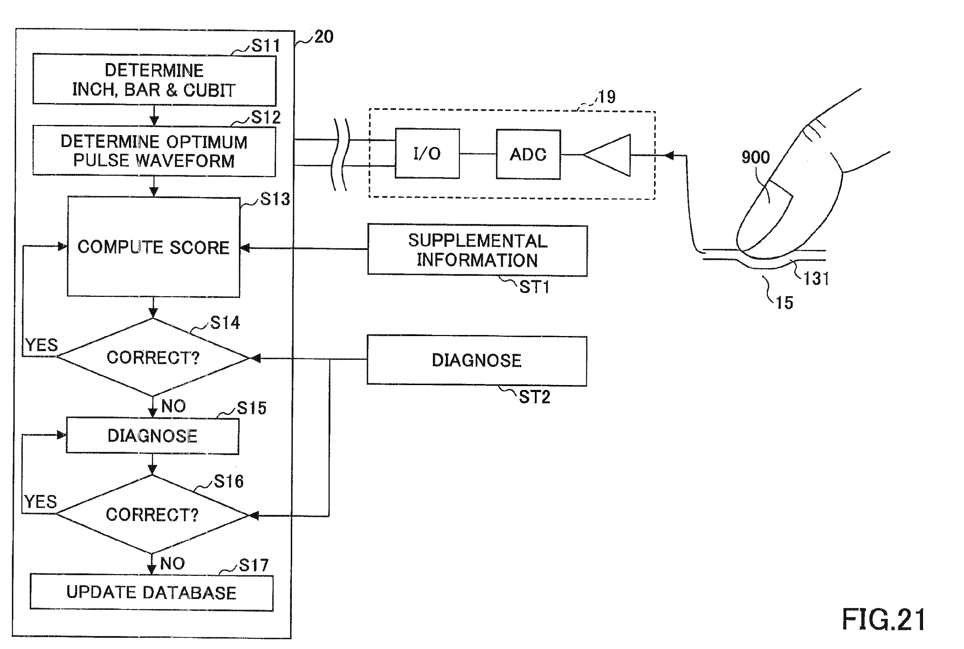

Next, a description will be given of a learning process of the database, by referring to FIG. 21. FIG. 21 is a diagram for explaining an example of the learning process of the database. In FIG. 21, those parts that are the same as those corresponding parts in FIGs. 1 and 5 are designated by the same reference numerals, and a description thereof will be omitted. In FIG. 21, steps S11 through S17 are executed by the CPU 20 interactively with the practitioner. Steps ST1 and ST2 are executed by the practitioner. In this example, the analog sensor output signal from the sensor 131 is input to the computer 20 via the signal processing circuit 19. The signal processing circuit 19 subjects the analog sensor output signal to a signal processing such as amplification, filtering, ADC, or the like, and inputs a digital sensor output signal to the I/F 25 (not illustrated) of the computer system 20 via a cable network, a wireless network, a cable-and-wireless combination network, or the like.

The computer system 20, in step S11, determines the XY-coordinate positions of the inch, bar, and cubit of both the patient's right and left hands, similarly to step S4 illustrated in FIG. 11, and in step S12, determines the optimum pulse waveforms at the determined XY-coordinate positions of the inch, bar, and cubit of both the patient's right and left hands, similarly to step S4 illustrated in FIG. 11. In step S13, the scores are computed with respect to the pulses at the total of 6 observation positions at the inch, bar, and cubit of both the patient's right and left hands, similarly to step S5 illustrated in FIG. 11. When computing the scores in step S13, the practitioner in step ST1 may input, from the input part 23, supplemental information related to the patient obtained by tongue inspection, abdominal examination, inquiry, or the like, for example. In this case, the computed scores may be corrected, categorized, or the like, based on the supplemental information.

In step S14, the scores at the total of 6 observation positions are integrated, and the patient's state is analogized by referring to the database based on the integrated score, similarly to step S6 illustrated in FIG. 11. When analogizing the patient's state in step S14, the practitioner in step ST2 may input, from the input part 23, analogized results made by the practitioner himself. In this case, the analogized result obtained from the database may be corrected based on the analogized results input by the practitioner himself. In addition, in a case in which a plurality of analogized results are obtained in step S13, step S14 may enable the practitioner, in step ST2, to operate the input part 23 and select one analogized result from amongst the plurality of analogized results displayed on the display part 24. In the case in which the analogized result is corrected in step S14, the process returns to step S13, and the computation, categorization, or the like of the score are performed again.

In step S15, the diagnostic result is generated based on the analogized result and displayed on the display part 24, for example, similarly to step S7 illustrated in FIG. 11. The diagnostic result may include the score, the patient's abnormal solid and hollow viscera (internal organ), human constitution, pathological condition (pattern), therapeutic effect (prognosis), or the like at the total of 6 observation positions. In step S16, a judgment is made to determine whether to correct the diagnostic result. The practitioner in step ST2 may input, from the input part 23, for example, diagnostic results made by the practitioner himself. In this case, the generated diagnostic result may be corrected based on the diagnostic results input by the practitioner himself. In addition, in a case in which a plurality of diagnostic results are obtained in step S15, step S16 may enable the practitioner, in step ST2, to operate the input part 23 and select one diagnostic result from amongst the plurality of diagnostic results displayed on the display part 24. In the case in which the diagnostic result is corrected in step S16, the process returns to step S15, and the generation of the diagnostic result is performed again.

In step S17, the diagnostic result is stored in the storage part 22, for example, as a part of the electronic medial record, so as to update the database of the electronic medial record.

In a case in which the sensor 131 is sufficiently thin (for example, 100 µm or less) such that the pulse can be transmitted to the practitioner's hand, and is sufficiently soft for attaching the sensor 131 to the patient's hand 15, the practitioner can sense the patient's pulse through the sensor 131. In this case, the acquisition of the pulse waveform by the sensor 131 and the palpation by the practitioner can be performed simultaneously, and the practitioner's diagnosis made by applying pressure can be digitized. That is, because the pulse is transmitted to the practitioner's hand through the sensor 131, both the sensation at the practitioner's fingertips and the electrical signal of the pulse waveform are obtained. Hence, the practitioner can simultaneously sense useful information from the practitioner's fingertips, and record the practitioner's diagnostic conditions (pressure applying positions, pressures, or the like) and the biometric reactions (pulse, tension, pressure, or the like) indicated by the pulses by converting the practitioner's diagnostic conditions and the biometric reactions into electrical signals.

In other words, an operation of sensing the pulses by placing the practitioner's fingertips on the patient's arteria radialis and finding the positions of the inch, bar, and cubit, can be performed through the sensor 131. Hence, the practitioner can confirm the accuracy or the like of the practitioner's operation by comparing the positions of the inch, bar, and cubit determined by the diagnostic apparatus and the positions of the inch, bar, and cubit sensed by the practitioner's fingertips. In addition, an operation applying an optimum pressure (pressure at which the pulse can be sensed to a maximum) on the patient's arteria radialis by the practitioner's fingertips and sensing the pulses can be performed through the sensor 131. Hence, the practitioner can confirm the accuracy or the like of the practitioner's operation by comparing the optimum pulse waveform determined by the diagnostic apparatus and the pressure sensed by the practitioner's fingertips. Accordingly, the diagnostic apparatus can be used for teaching, to the practitioner, the operation of determining, by hand, the positions of the inch, bar, and cubit, and the operation of determining, by hand, the optimum pulse waveform at the inch, bar, and cubit.

According to the above embodiment, the palpation based on the pulse can be performed without being greatly dependent upon the practitioner. In addition, the positions of the inch, bar, and cubit, and the optimum pulse waveforms at the inch, bar, and cubit can be determined without being dependent upon the practitioner, and it is also possible to cope with individual differences of the patient.

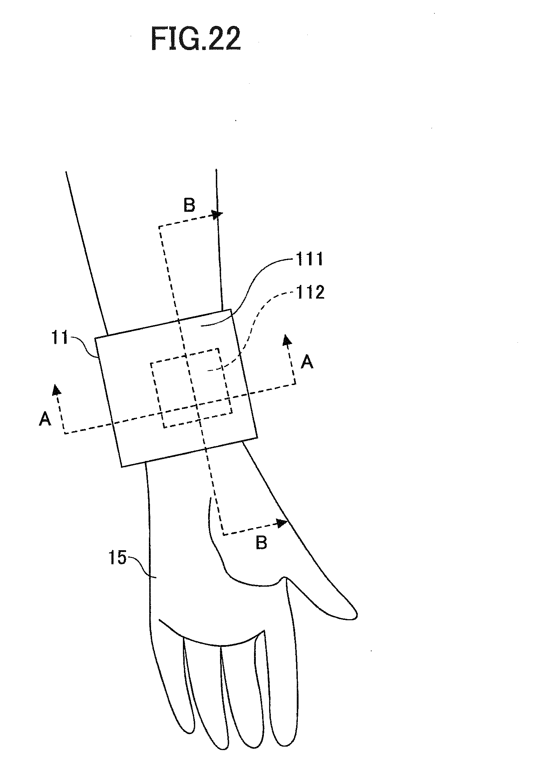

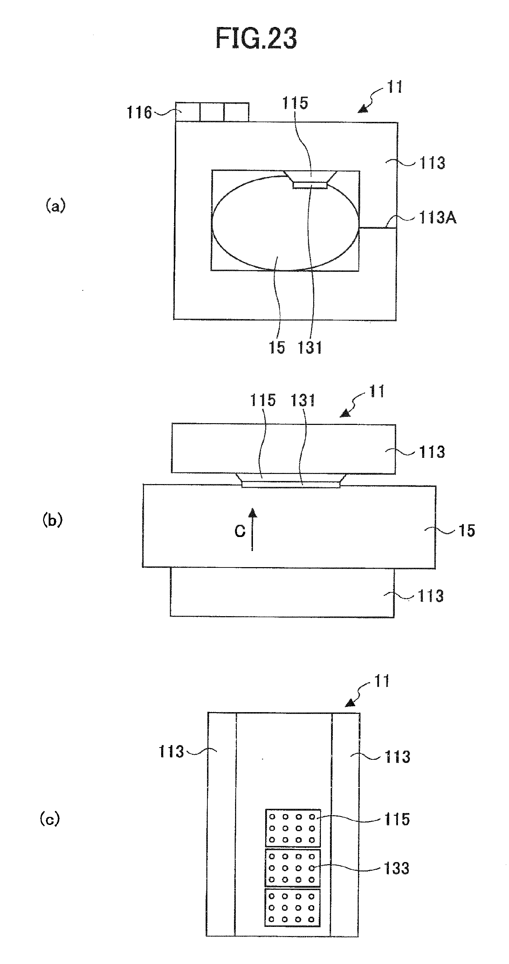

Next, a description will be given of a configuration of a sensor part that mechanically applies pressure on the patient's hand, by referring to FIGs. 22 and 23. FIG. 22 is a diagram illustrating an example of the sensor part attached to the patient's hand. FIG. 23 is a diagram for explaining the configuration of the sensor part illustrated in FIG. 22.

In FIG. 22, the sensor part 11 is attached to a part including the inch, bar, and cubit of the patient's hand 15. The sensor part 11 includes a pressure applying part 111. The pressure applying part 111 applies pressure on a pressure applying region 112.

In FIG. 23, (a) is a cross sectional view of the sensor part 11 along a line A-A in FIG. 22, (b) is a cross sectional view of the sensor part 11 along a line B-B in FIG. 22, and (c) is a diagram viewed along a direction C in (b). As illustrated in FIG. 23, the sensor part 11 includes a belt 113 that can be fastened at a connecting part 113A, and is fastened around the patient's hand (arm) 15. The belt 113 includes an airbag 115, and a sensor 131 mounted on the airbag 115. The airbag 115 may have any configuration for independently applying pressure at 3 regions corresponding to the inch, bar, and cubit, and may be formed by 3 separate airbags. Air is injected into the airbag 115 by a pump 116 that is provided on the belt 113, and controls the pressure applied on the hand 15. The pump 116 may have 3 separate pump parts capable of independently injecting air into 3 regions of the airbag 115 respectively corresponding to the inch, bar, and cubit. The pump 116 is controllable by the CPU 21, and the CPU 21 can independently control the pressure applied to the hand 15 at the 3 regions of the airbag 115. The airbag 115 and the pump 116 form an example of a pressure applying means.

The pump 116 may be separate from the belt 113 and configured to be externally connected with respect to the belt 113. In this case, the air from the pump 116 may be supplied to the airbag 115 through a tube that is connected to the belt 113, for example.

The pressure applying means is not limited to the airbag 115 and the pump 116. For example, a mechanism for directly applying pressure on the hand by a pump or the like may be used for the pressure applying means. The pressure applying means may be any means capable of mechanically applying pressure on the patient's hand 15.

By using the means for mechanically applying pressure on the patient's hand as illustrated in FIGs. 22 and 23, the computer system 20 can automatically perform the operation of determining the positions of the inch, bar, and cubit, and the operation of determining the optimum pulse waveform at the inch, bar, and cubit, without troubling the practitioner.

Further, although the diagnostic apparatus, the diagnostic method, and the program disclosed herein are described by way of embodiments, the present invention is not limited to these embodiments, and various variations and modifications may be made without departing from the scope of the present invention.

### DESCRIPTION OF THE REFERENCE NUMERALS

- 11: Sensor Part
- 20: Computer System
- 21: CPU
- 22: Storage Part
- 23: Input Part
- 24: Display Part
- 25: I/F
- 26: Bus
- 131: Sensor

## Claims

1. A diagnostic apparatus **characterized in that** there are provided:
a sensor part having a plurality of sensor cells provided in a matrix arrangement on a sensor that detects a pulse within a region in which the pulse of a diagnostic target is detected, in a state in which a pressure is applied to the region through the sensor;
means for acquiring a distribution of a pulse waveform, based on a sensor signal indicating the pulse detected by the sensor part in a state in which the pressure is constant;
means for determining a plurality of observation positions within the region, and determining a pulse waveform having a maximum amplitude at the plurality of observation positions that are determined by increasing the pressure, based on the distribution of the pulse waveform;
means for computing a digitized score of the pulse at each observation position, based on the pressure at a time when the pulse waveform having the maximum amplitude is obtained at each observation position;
means for analogizing a state of the diagnosis target, by integrating scores at the plurality of observation positions, and referring to a database that prestores analogized states of the diagnosis target with respect to the scores, based on an integrated score; and
means for generating a diagnostic result with respect to the diagnostic target from the analogized state of the diagnostic target, and outputting the diagnostic result.

2. The diagnostic apparatus as claimed in claim 1, **characterized in that** the plurality of observation positions are an inch, a bar, and a cubit of arteria radialis.

3. The diagnostic apparatus as claimed in claim 1 or 2, **characterized in that** the means for computing computes the score with respect to pulse signs including a depth of pulse, an amplitude of pulse, a period of pulse, length and width of pulse, fluency of pulse, and tonus of pulse, according to a predetermined algorithm.

4. The diagnostic apparatus as claimed in claim 3, **characterized in that** the state of the diagnostic target analogized with respect to the score of the pulse sign, prestored in the database, is at least one of abnormal solid and hollow viscera, human constitution, pathological condition, illness, and therapeutic effect according to theories of oriental medicine.

5. The diagnostic apparatus as claimed in any of claims 1 to 4, **characterized in that** the sensor includes a flexible sheet, and the plurality of sensor cells are provided on the sheet.

6. The diagnostic apparatus as claimed in any of claims 1 to 5, **characterized in that** the sensor part includes a pressure applying means for applying the pressure.

7. The diagnostic apparatus as claimed in claim 6, **characterized in that** the pressure applying means includes an airbag.

8. A diagnostic method to be executed by a computer, **characterized in that** the computer is caused to execute:
a procedure for inputting a sensor signal from a sensor part having a plurality of sensor cells provided in a matrix arrangement on a sensor that detects a pulse within a region in which the pulse of a diagnostic target is detected, in a state in which a pressure is applied to the region through the sensor;
a procedure for acquiring a distribution of a pulse waveform, based on the sensor signal indicating the pulse detected by the sensor part in a state in which the pressure is constant;
a procedure for determining a plurality of observation positions within the region, and determining a pulse waveform having a maximum amplitude at the plurality of observation positions that are determined by increasing the pressure, based on the distribution of the pulse waveform;
a procedure for computing a digitized score of the pulse at each observation position, based on the pressure at a time when the pulse waveform having the maximum amplitude is obtained at each observation position;
a procedure for analogizing a state of the diagnosis target, by integrating scores at the plurality of observation positions, and referring to a database that prestores analogized states of the diagnosis target with respect to the scores, based on an integrated score; and
a procedure for generating a diagnostic result with respect to the diagnostic target from the analogized state of the diagnostic target, and outputting the diagnostic result.

9. The diagnostic method as claimed in claim 8, **characterized in that** the plurality of observation positions are an inch, a bar, and a cubit of arteria radialis.

10. The diagnostic method as claimed in claim 8 or 9, **characterized in that** the procedure for computing computes the score with respect to pulse signs including a depth of pulse, an amplitude of pulse, a period of pulse, length and width of pulse, fluency of pulse, and tonus of pulse, according to a predetermined algorithm.

11. The diagnostic method as claimed in claim 10, **characterized in that** the state of the diagnostic target analogized with respect to the score of the pulse sign, prestored in the database, is at least one of abnormal solid and hollow viscera, human constitution, pathological condition, illness, and therapeutic effect according to theories of oriental medicine.

12. The diagnostic method as claimed in any of claims 8 to 11, **characterized in that**:
the sensor part includes a pressure applying means for applying the pressure, and
the computer is caused to further execute a procedure for controlling the pressure applying means.

13. A program for causing a computer to execute a diagnostic process, **characterized in that** the diagnostic process comprises:
a procedure for inputting a sensor signal from a sensor part having a plurality of sensor cells provided in a matrix arrangement on a sensor that detects a pulse within a region in which the pulse of a diagnostic target is detected, in a state in which a pressure is applied to the region through the sensor;
a procedure for acquiring a distribution of a pulse waveform, based on the sensor signal indicating the pulse detected by the sensor part in a state in which the pressure is constant;
a procedure for determining a plurality of observation positions within the region, and determining a pulse waveform having a maximum amplitude at the plurality of observation positions that are determined by increasing the pressure, based on the distribution of the pulse waveform;
a procedure for computing a digitized score of the pulse at each observation position, based on the pressure at a time when the pulse waveform having the maximum amplitude is obtained at each observation position;
a procedure for analogizing a state of the diagnosis target, by integrating scores at the plurality of observation positions, and referring to a database that prestores analogized states of the diagnosis target with respect to the scores, based on an integrated score; and
a procedure for generating a diagnostic result with respect to the diagnostic target from the analogized state of the diagnostic target, and outputting the diagnostic result.

14. The program as claimed in claim 13, **characterized in that** the plurality of observation positions are an inch, a bar, and a cubit of arteria radialis.

15. The program as claimed in claim 13 or 14, **characterized in that** the procedure for computing computes the score with respect to pulse signs including a depth of pulse, an amplitude of pulse, a period of pulse, length and width of pulse, fluency of pulse, and tonus of pulse, according to a predetermined algorithm.

16. The program as claimed in claim 15, **characterized in that** the state of the diagnostic target analogized with respect to the score of the pulse sign, prestored in the database, is at least one of abnormal solid and hollow viscera, human constitution, pathological condition, illness, and therapeutic effect according to theories of oriental medicine.

17. The program as claimed in any of claims 13 to 16, **characterized in that**:
the sensor part includes a pressure applying means for applying the pressure, and
the computer is caused to further execute a procedure for controlling the pressure applying means.
